# EUROPEAN PATENT APPLICATION

(11) **EP 0 577 224 A1**
(43) Date of publication of application: **05.01.1994**
(21) Application number: 93202308.8
(22) Date of filing: 14.04.1987
(51) Int. Cl.: C07K 15/04, A61K 39/09, G01N 33/569

(54) **Bacterial antigens, antibodies, vaccines and methods of manufacture**

(30) Priority: 16.04.1986 US 852840
(62) Divisional of application: 87903113.6
(71) Applicant: THE BRIGHAM AND WOMEN'S HOSPITAL, INC., Boston, MA 02115 (US)
(72) Inventor: Kasper, Dennis L., Newton Center MA 02159 (US); Jennings, Harold J., Gloucester, Ontario K1J 6G6 (CA); Levy, Nancy J., Newton Center MA 02159 (US); Wessels, Michael R., Brookline MA 02146 (US)
(74) Representative: Allard, Susan Joyce

(57) **Abstract**

A protein as described which is a substantially purified trypsin-resistant C surface protein of type I/c Group B Streptococcus which has a molecular weight of about 14,000 and which is non-cross-immunoreactive with group B Streptococcus bacterial polysaccharides, yet cross-immunogenic with type Ia/c Group B Streptococcus (GBS). The protein or a fragment comprising an immunodeterminant thereof is used in a vaccine that elicits protection against type Ia/c GBS, the protein or fragment being optionally conjugated to a carrier.

## Description

This invention relates to antigens having immunogenic determinants of Group B Streptococcus bacteria, to vaccines protecting against those bacteria and to methods of making such vaccines. As used in this application, the term Group B Streptococcus (of GBS) bacteria is used as understood by those in the field, particularly with reference to Lancefield, J. Exp. Med. 108:329-341 (1938) and subsequent work further characterizing Group B serotypes, e.g. Russell-Jones, J. Exp. Med. 160:1476 (1984). The term specifically includes bacteria taxonomically designated Streptococcus agalactiae.

GBS are a recognized etiological agent for bacteremia and/or meningitis in infants, and for infections in adults. Baker, "Group B Streptococcal Infections" in Advances in Internal Medicine, 25:475-500 (1980). Accordingly, it is important to develope rapid and definitive assays for diagnosis of GBS infection, and methods of generating protection against GBS, particularly in infants and compromised individuals.

Studies of GBS protein immunogenicity have been reported. Lancefield et al., J. Exp. Med. 142:165-179 (1975) report that the so-called C proteins of GBS are capable of inducing protective antibodies when present as an immunogen on a whole organism. The nomenclature "C proteins" is used as defined by Henrichsen et al., Int. J. of Systematic Bacteriol. 34:500 (1984), and the term includes proteins formerly designated Ibc proteins.

Molecular studies of C proteins have shown that these substances constitute a complex group of proteins of variable molecular weight. Wilkinson et al., Infect. Immunol. 4:596-604 (1971); Bevanger et al., Acta Path. Microbiol. Scand. Sect. B, 87:51-54 (1979); Russell-Jones et al., J/ Exp. Med. 160:1476-1484 (1984). Bevanger et al., Acta Path. Microbiol. Scand. Sect. B, 89:205-209 (1981).

Wilkinson et al. disclose that hot acid extracts of whole type Ic GBS yields 13 proteins of varying molecular size (determined by polyacrylamide gel electrophoresis) having serological reactivity in agar gel against type Ic antisera. Bevanger et al. (1979) disclose GBS acid extract containing nine trypsin sensitive proteins and five trypsin resistant but pepsin sensitive proteins. Bevanger et al. (1981) disclose immunogenicity of partially purified acid extracted proteins. Russell-Jones et al. disclose extracts containing up to 30 proteins varying in molecular weight from 20,000 to 130,000, the 130 kd protein being predominant. Russell-Jones et al. also report that multiple proteins reacted by immuno (Western) blot to a single monoclonal antibody, suggesting that some proteins were breakdown products of others. Using another monoclonal antibody, the 130,000 MW proteins and 12 other proteins spaced between 10,000 and 120,000 MW appeared to share a common epitope.

In accordance with the present invention there is provided a substantially purified trypsin-resistant C-surface protein of type I/c Group B Streptococcus which has a molecular weight of about 14,000 and which is non-cross-immunoreactive with group B Streptococcus bacterial polysaccharide, yet cross-immunogenic with type Ia/c group B Streptococcus (GBS).

The protein may elicit protection against type Ia/c GBS and be cross-immunoreactive with type Ib/c group B Streptococcus bacteria.

"Purified" means substantially separated from the various lipid and carbohydrate components that may naturally occur with the protein.

In another aspect of the invention the above described protein or a fragment comprising an immunodeterminant thereof, is used in a vaccine that elicits protection against type Ia/c (and type Ib/c) GBS, which vaccine comprises the protein or fragment thereof optionally conjugated to a carrier such as an oligosaccharide which is not an oligosaccharide of the formula: 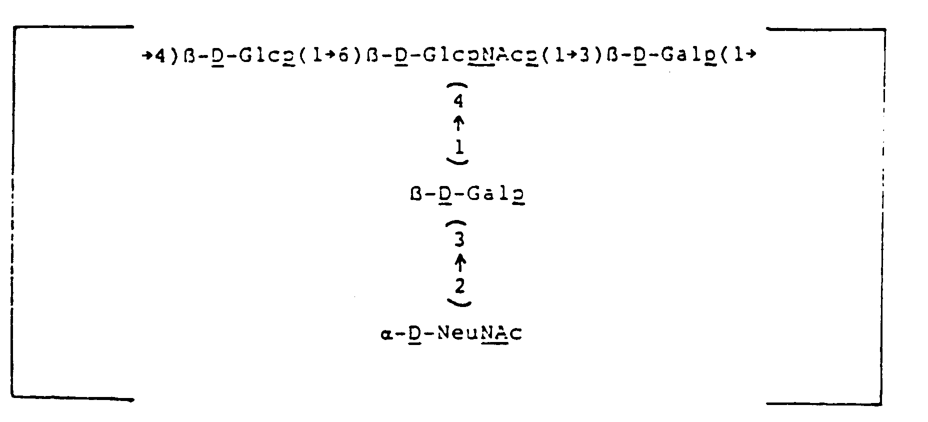
and a pharmaceutically acceptable vehicle.

In yet another aspect, the invention features a gamma globulin fraction capable of passive protection against GBS, the fraction being produced by immunizing a mammal with the above-described vaccine. The fraction is then administered to an individual to provide protection against GBS infection or to treat on-going infection.

Finally, the invention features a method of assaying a sample for anti GBS antibody by adding to the sample the protein antigen described above, and then detecting the formation of an immunocomplex; alternatively a sample is assayed for the presence of a GBS immunodeterminant by raising an antibody to the protein antigen, adding the antibody to the sample, and detecting the formation of an immunocomplex.

We now describe a preferred embodiment of the invention.

### I. GBS Protein Antigen

An antigenic GBS C protein is isolated from type Ic GBS culture supernatants as illustrated by the following example. The protein is immunologically reactive with antisera (e.g., mouse antisera) to a P10 GBS fraction, i.e., a fraction including proteins of MW between 10,000 and 30,000. The purified protein elicits rabbit antisera that protect mice against challenge with type Ib GBS. This protective ability is not affected by incubations with GBS polysaccharides.

The following examples illustrate the protein antigen, the method of isolating it and the method of passive immunization.

### Example 1: Production and purification of protein antigens

Type Ia/c GBS (e.g. Channing Laboratory Strain A909 Rockefeller University N.Y., N.Y., ATCC 27591) are cultured into late log phase in dialysate of Todd Hewett Broth (THB, Difco Laboratories, Detroit, MI), and then resuspended in 0.3% formalin in 0.15M sodium phosphate buffer (pH7.4), by the general method of Levy et al. J. Infect Dis. 149:851-868 (1984).

A one liter log phase culture of type Ic is added to 15 liters of the dialusate of THB (dialyzed on an exclusion membrane of 10,000 MW), supplemented with 10% glucose, and grown into late log phase maintaining neutral pH by titration in a fermentor (Biolafitte Model BL 20.2, LSL Biolafitte Inc., Princeton, NJ). After removal of the bacteria by pelleting at 10,000 g, the supernatant is concentrated to 100 ml on a Pellicon apparatus (Millipore Corp., Bedford, MA) containing a 10,000 MW exlusion membrane, dialyzed against H₂O extensively and frozen at -20^{o}C.

The culture supernatant is separated by approximate molecular size using ultrafiltration. Two fractions are obtained, one retained by a PM30 membrane (P30; greater than 30,000 MW), and another retained by a PM10 membrane but not retained by a PM30 (P10; greater than 10,000 MW and less than 30,000 MW). The P10 is dialyzed against 0.05 M sodium phosphate buffer, pH 7.4 with 0.15 M NaCl and fractionated on a Sephadex G-75 column (1.6 x 82 cm) (Pharmacia). The protein content of fractions is monitored by absorption at 750 nm in the Lowry assay, J. Biol. Chem, 193:265-275 (1951).

### Example 2: SDS-PAGE and Western blot

Samples are analyzed by SDS-PAGE following the method of Laemmli, Nature 227:680-684 (1970). Gradient gels either 5-15% or 10-20% are employed depending on the molecular weight of the proteins. Samples for electrophoresis are diluted in a buffer containing 0.1 M Tris, pH8, 1% SDS, 4 M urea and 60 mM iodoacetamide as final concentration. When reduction of the samples is required, 50 mM dithiothreiotol is included in the buffer. One hundred µ1 samples containing 100 µg protein are placed into each well. After electrophoresis the proteins are visualized by Coomassie brilliant blue satin.

To identify immunologically reactive antigens, Western blot is used. Proteins are first subjected to electrophoresis and the SDS-PAGE gel washed in Buffer A composed of 0.02 M Tris buffer, pH 8.5 with 0.15 M glycine and 20% methanol. The proteins are transferred to nitrocellulose sheets (Schleicher and Schuell, Keene, NH) by electrophoresis (150 mA, 20 h) in Buffer A and washed in 0.01 M Tris buffer, pH 7.4 with 0.5 M NaCl and 0.1% Brig 58 (Buffer B). The sheet is then incubated with antiserum for 2 h at 22^{o}C and washed in Buffer B. After incubation with ¹²⁵I-protein A for 30 min at 22^{o}C, the sheet is washed in Buffer B, blotted dry and exposed to Kodak XAR 5 film.

### Example 3: Mouse protection studies

Male outbred CD-1 mice weighing 22-24 gr are obtained from Charles River Breeding Laboratories (Wilmington, MA). Sera obtained from the mice contained no detectable antibody to type Ia/c GBS as tested by RABA. Each experimental group contained between 5 and 10 animals. For mouse protection studies, mice are injected IP with 1 ml mouse or rabbit antiserum and 24 h later challenged IP with 1 x 10⁶ CFU of type Ib/c GBS. Preimmune rabbit or mouse serum is used as the control. Mice are examined every 24 h and the number of surviving mice is calculated 48 h after bacterial challenge.

For absorption of protective antibodies from antisera, protein fractions in concentrations of 1 mg per ml are used. One ml of antiserum and one ml of protein is incubated for 2 h at 37^{o}C and then overnight at 4^{o}C. The mixture is then pelleted at 13,000 g for 3 min and the supernatant is diluted in 0.15 M NaCl for protection studies.

After partial purification by column chromatography, the 14,000 MW protein was reisolated from preparative SDS-PAGE gel and used to elicit antiserum in a rabbit. In mouse protection studies this rabbit antiserum protected mice (98%) against Ia/c GBS challenge.

To investigate whether the P10 fraction could elicit mouse protective antibodies against a heterologous GBS strain, mice were immunized with the type Ia/c P10 fraction. The post immunization antisera were then pooled and examined for its capacity to protect mice against challenge with type Ib GBS. Mice were injected IP with various dilutions of pooled mouse antisera to the P10 fraction followed 24 h later by challenge with the type Ib/c strain. The protective capacity of the sera begins to dilute out at a 1:80 dilution of the sera. Therefore the sera were used at an optimal dilution of 1:40 for these experiments. In mouse protection studies the immune sera protected mice against challenge with the type Ib strain (P < 0.001) compared with the normal mouse sera at the same dilution. This pooled sera contained no detectable antibody to the type Ia polysaccharide as measured in the RABA. Furthermore absorption of the pooled antisera with the type Ia or Ib polysaccharide did not significantly alter the protective level. There was protective efficacy of mouse antisera to P10 fraction from type Ic which contained non-capsular antigens which elicited antibody in mice, the antisera passively protecting mice against challenge with the type Ib GBS.

Specifically, rabbit antiserum to the SDS-PAGE eluted 14,000 MW protein from type Ic GBS was next used in mouse protection studies. Mice were injected with antiserum IP and challenged 24 hours later with type Ib GBS. The antiserum elicited to the SDS-PAGE eluted 14,000 MW protein was protective in mice at dilutions of 1:5 and 1:10. The difference between the normal rabbit serum and the antiserum elicited to the SDS-PAGE eluted 14,000 MW protein was significant.

### II. Protein-oligosaccharide conjugation

For the preparation of vaccines conjugation of the protein of the invention to a carrier such as an oligosaccharide is useful.

Protein-oligosaccharide conjugation is achieved by a coupling reaction, such as the general method of Swartz and Gray, Arch. Bioch. Biophys. (1977) 181:542, which couples a reducing sugar directly to a protein. Oligosaccharide fragments produced by deamination can be directly linked via cyanoborhydride to the carrier protein. In the case of III GBS as mentioned above, a secondary amine is formed by bonding between a nitrogen on the protein and an aldehyde group on 2,5-anhydromannose formed by deamination of the oligosaccharide. This aldehyde is available for direct coupling using cyanoborhydride.

The resulting oligosaccharide-protein conjugate is suspended in pyrogen-free saline or any other physiologically acceptable non-toxic vehicle or medium, which may contain any conventional stabilizers or other additives as desired.

### III. Use in Passive Immunization

The protein of the invention is also useful in a method of passive immunization, used particularly for infants or compromised adults, by injecting the oligosaccharide-protein conjugate vaccine into a human to raise antibodies thereto in high titer, separating the antiserum from the blood of the human, and fractionating the antiserum to produce a gamma globulin fraction containing the antibodies, which can be used for passive immunization. This method of establishing donors for passive immunization is useful because, although occasional non-immunized human adults have very high levels of type-specific GBS antibody in their sera, it would be necessary to screen very large populations to select those individuals whose plasma could be pooled to make sufficiently high titered globulin fractions to be useful for passive immunizations. Several lots of pooled human gamma globulin from non-immunized adults have been assayed for anti-Types Ia and Ic antibody and have been found to contain only 5-20 µg/ml of serum. Immunizations by intravenous injection of such low titer globulins would require prohibitively large doses with attendant risk of adverse reactions.

## Claims

1. A substantially purified trypsin-resistant C surface protein of type Ic group B Streptococcus which has a molecular weight about 14,000 and which is non-cross-immunoreactive with group B Streptococcus bacterial polysaccharides yet cross-immunogenic with type Ia/c Group a Streptococcus.

2. The protein of claim 1 which elicits protection against type Ia/c GBS and which is cross-immunoreactive with type Ib/c Group B Streptococcus bacteria.

3. A vaccine that elicits protection against type Ia/c Group B Streptococcus bacteria comprising a pharmaceutically acceptable vehicle and an immunogen comprising the protein of claim 1 or claim 2 or a fragment comprising an immunodeterminant thereof.

4. A vaccine as claimed in claim 3 wherein said protein or fragment is conjugated to a carrier.

5. A vaccine as claimed in claim 4 wherein the carrier is an oligosaccharide other than that of the formula:

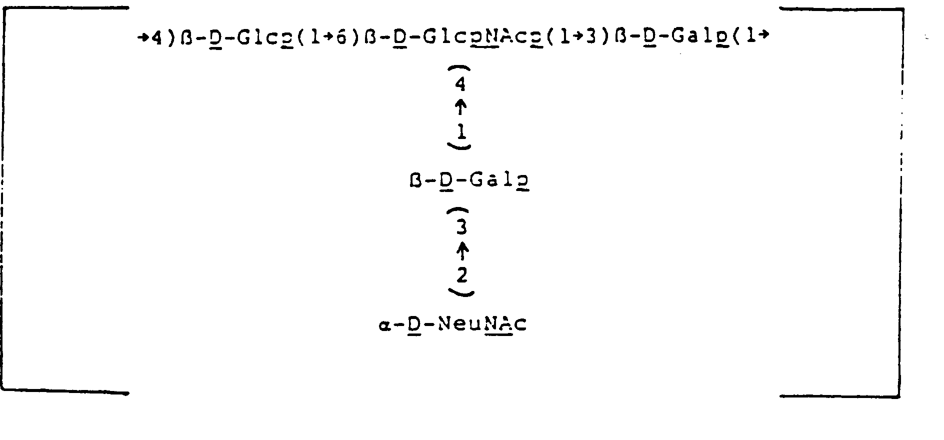

6. A gamma globulin fraction capable of passive protection against GBS, said gamma globulin fraction being produced by immunizing a mammal with the vaccine of any one of claims 3 to 5, and recovering gamma globulin from said individual.

7. A method of assaying a sample for the presence of any GBS antibody comprising adding to said sample the protein of claim 1 or claim 2 and detecting the formation of an immunocomplex.

8. A method of assaying a sample for the presence of a GBS immunodeterminant comprising raising an antibody to the protein of claim 1 or claim 2, adding said antibody to said sample and detecting the formation of an immunocomplex.
